**Europäisches Patentamt**

**European Patent Office**

**Office européen des brevets**

(19)

(11) Veröffentlichungsnummer: **0 284 971 B1**

(12) **EUROPÄISCHE PATENTSCHRIFT**

(45) Veröffentlichungstag der Patentschrift: **21.08.91**

(21) Anmeldenummer: **88104604.9**

(22) Anmeldetag: **23.03.88**

(51) Int. Cl.⁵: **C07C 11/167**, C07C 7/08, C07C 7/04

(54) **Verfahren zur Gewinnung von 1,3-Butadien.**

(30) Priorität: **28.03.87 DE 3710434**

(43) Veröffentlichungstag der Anmeldung:
**05.10.88 Patentblatt 88/40**

(45) Bekanntmachung des Hinweises auf die
Patenterteilung:
**21.08.91 Patentblatt 91/34**

(84) Benannte Vertragsstaaten:
**AT BE DE ES FR GB IT NL SE**

(56) Entgegenhaltungen:
**FR-A- 2 413 347**
**US-A- 2 707 716**
**US-A- 3 686 349**
**US-A- 3 772 158**

**PATENT ABSTRACTS OF JAPAN, Band 10,
Nr. 17 (C-324) (2074), 23. januar 1986; JP-A-60
174 733 (NIPPON GOSEI GOMU), 09.09.1985**

(73) Patentinhaber: **BASF Aktiengesellschaft
Carl-Bosch-Strasse 38
W-6700 Ludwigshafen(DE)**

(72) Erfinder: **Kaibel, Gerd, Dr.
Robert-Bosch-Strasse 4
W-6840 Lampertheim(DE)**
Erfinder: **Hefner, Werner, Dr.
In der Teichgewann 20
W-6840 Lampertheim(DE)**
Erfinder: **Keller, Peter, Dr.
Brunengasse 24
W-6940 Weinheim(DE)**
Erfinder: **Drewitz, Werner
Im Eichgarten 51
W-6701 Erpolzheim(DE)**

## Beschreibung

Die vorliegende Erfindung betrifft ein Verfahren zur Gewinnung von 1,3-Butadien aus einem 1,3-Butadien und geringe Mengen Propin und $C_5$-Kohlenwasserstoffe enthaltenden $C_4$-Kohlenwasserstoffgemisch durch Extraktivdestillation mittels eines selektiven Lösungsmittels und anschließende destillative Reinigung des bei der Extraktivdestillation erhaltenen Roh-1,3-Butadiens.

Es ist bekannt, 1,3-Butadien in der Weise zu gewinnen, daß zunächst aus einem 1,3-Butadien enthaltenden $C_4$-Kohlenwasserstoffgemisch durch Extraktivdestillation mittels eines selektiven Lösungsmittels ein Roh-1,3-Butadien erhalten wird. Das Roh-1,3-Butadien, das für viele Anwendungszwecke, z.B. für Polymerisationen, nicht hinreichend rein ist, wird anschließend einer destillativen Reinigung in zwei konventionellen Destillationsschritten unterworfen, wobei das Roh-1,3-Butadien zunächst in flüssiger Form einer ersten Distillationskolonne zugeführt wird, in der die leichtersiedenden Verunreinigungen wie Propin im Kopfprodukt abgezogen werden. 1,3-Butadien und die höhersiedenden Verunreinigungen wie die $C_5$-Kohlenwasserstoffe, 1,2-Butadien und gegebenenfalls cis-2-Buten werden als Sumpfprodukt entnommen und einer zweiten Destillationskolonne zugeführt, in der 1,3-Butadien als Kopfprodukt erhalten wird, während die höhersiedenden Verunreinigungen im Sumpfprodukt abgezogen werden.

Die erste Destillationskolonne wird bei dem bekannten Verfahren bei erhöhtem Druck betrieben, der erforderlich ist, um das größere Mengen Propin enthaltende Kopfprodukt bei einer Mindesttemperatur kondensieren zu können, die es ermöglicht, die Kondensationswärme an Kühlwasser üblicher Temperatur, z.B. Flußwasser, oder die Umgebungsluft abzuführen. Niedrigere Destillationsdrucke würden eine Kühlung mit Kaltwasser oder einem Kühlmittel, z.B. Sole, erfordern und zu wesentlich höheren Betriebskosten führen. Bei der zweiten Destillationskolonne liegt als Kopfprodukt das reine 1,3-Butadien mit einem höheren Siedepunkt vor, so daß der Betriebsdruck der zweiten Destillationskolonne niedriger gewählt werden kann.

Das bekannte Verfahren hat jedoch den Nachteil, daß die Energieverbräuche, insbesondere bei der destillativen Reinigung des Roh-1,3-Butadiens, noch nicht zufriedenstellend sind.

Die vorliegende Erfindung soll nun eine Verbesserung der Arbeitsweise und insbesondere Wirtschaftlichkeit der bekannten Verfahren bewirken.

Es wurde nun ein vorteilhaftes Verfahren gefunden, zur Gewinnung von 1,3-Butadien aus einem 1,3-Butadien und geringe Mengen Propin und $C_5$-Kohlenwasserstoffe enthaltenden $C_4$-Kohlenwasser-

stoffgemisch, wobei man zunächst durch Extraktivdestillation mittels eines selektiven Lösungsmittels ein Roh-1,3-Butadien abtrennt, aus dem durch anschließende Destillation unter Abtrennung eines die leichter als 1,3-Butadien siedenden Kohlenwasserstoffe (im folgenden als "leichtersiedende Verunreinigungen" bezeichnet) enthaltenden Stromes und eines die höher als 1,3-Butadien siedenden Kohlenwasserstoffe (im folgenden als "höhersiedende Verunreinigungen" bezeichnet) enthaltenden Stroms ein 1,3-Butadien hoher Reinheit erhalten wird, welches dadurch gekennzeichnet ist, daß die Destillation des Roh-1,3-Butadiens in zwei zusammengeschalteten Destillationszonen durchgeführt wird, wobei man

a) das Roh-1,3-Butadien dampfförmig der ersten Destillationszone zuführt,

b) aus der ersten Destillationszone ein Kopfprodukt, das 1,3-Butadien sowie leichtersiedende Verunreinigungen enthält, flüssig abzieht und dem mittleren Bereich der zweiten Destillationszone zuführt,

c) im Abtriebsteil der ersten Destillationszone einen flüssigen Seitenstrom, der 1,3-Butadien sowie höhersiedende Verunreinigungen enthält, abzieht und am Sumpf der zweiten Destillationszone zuführt.

d) am Sumpf der ersten Destillationszone die höhersiedenden Verunreinigungen abzieht,

e) am Kopf der zweiten Destillationszone die leichtersiedenden Verunreinigungen abzieht,

f) im Abtriebsteil der zweiten Destillationszone 1,3-Butadien als Produkt flüssig abzieht und

g) das Sumpfprodukt der zweiten Destillationszone, das 1,3-Butadien und höhersiedende Verunreinigungen enthält, flüssig abzieht und in die erste Destillationszone im Bereich der Entnahme des flüssigen Seitenstromes zurückführt.

Nach dem neuen Verfahren kann der Energieaufwand bei der destillativen Reinigung des Roh-1,3-Butadiens um etwa 25 % gegenüber dem bekannten Verfahren gesenkt werden. Gleichzeitig können bei der erfindungsgemäßen Zusammenschaltung der Destillationskolonnen, Kolonnen mit geringeren Abmessungen verwendet werden, so daß auch eine Senkung der Investitionskosten ermöglicht wird. Die geringeren Abmessungen der Destillationskolonnen ermöglichen es weiter, daß bei einer Umrüstung einer nach dem bekannten Verfahren betriebenen technischen Anlage zur Gewinnung von 1,3-Butadien auf das erfindungsgemäße Verfahren der Destillationsteil der umgerüsteten Anlage mit erhöter Kapazität betrieben werden kann.

Die erfindungsgemäß für die Gewinnung von 1,3-Butadien einzusetzenden, Propin und $C_5$-Kohlenwasserstoffe enthaltenden $C_4$-Kohlenwasserstoffgemische werden z.B. bei der Herstellung von Ethylen und/oder Propylen durch thermisches Spalten einer Petroleumfraktion, z.B. von verflüssigtem Petroleumgas (LPG), Leichtbenzin (Naphtha), Gäsol und dergleichen, als Kohlenwasserstofffraktion erhalten. Weiterhin werden solche $C_4$-Fraktionen bei der katalytischen Dehydrierung von n-Butan und/oder n-Buten erhalten. Das $C_4$-Kohlenwasserstoffgemisch enthält in der Regel Butane, n-Buten, Isobuten, 1,3-Butadien, Butenin, 1-Butin, 1,2-Butadien, cis-2-Buten, trans-2,Buten, Propin und $C_5$-Kohlenwasserstoffe. Der Gehalt der $C_4$-Kohlenwasserstoffgemische an Propin und den $C_5$-Kohlenwasserstoffen liegt in der Regel jeweils unter 1 Gew.-%, meistens jeweils unter 0,5 Gew.%. Das erfindungsgemäße Verfahren ist jedoch auch auf $C_4$-Kohlenwasserstoffgemische mit höheren Propin-Gehalten und/oder Gehalten an $C_5$-Kohlenwasserstoffen anwendbar.

Geeignete selektive Lösungsmittel für das erfindungsgemäße Verfahren sind z.B. Butyrolacton, Nitrile wie Acetonitril, Propionitril, Methoxypropionitril, Ketone wie Aceton, Furfurol, N-alkylsubstituierte niedere aliphatische Säureamide, wie Dimethylformamid, Diethylformamid, Dimethylacetamid, Diethylacetamid, N-Formylmorpholin, N-alkylsubstituierte cyclische Säureamide (Lactame) wie N-Alkylpyrrolidone, insbesondere N-Methylpyrrolidon. Im allgemeinen werden N-alkylsubstituierte niedere aliphatische Säureamide oder N-alkylsubstituierte cyclische Säureamide verwendet. Mit besonderem Vorteil werden von den Lösungsmitteln Dimethylformamid und insbesondere N-Methylpyrrolidon verwendet.

Es können jedoch auch als selektives Lösungsmittel Mischungen dieser Lösungsmittel untereinander, z.B. von N-Methylpyrrolidon mit Acetonitril, Mischungen dieser Lösungsmittel mit Colösungsmitteln wie Wasser und/oder tert.-Butylether, z.B. Methyl-tert.-butylether, Ethyl-tert.-butylether, Propyl-tert.-butylether, n- oder iso-Butyl-tert.-butylether, eingesetzt werden.

Gemäß dem erfindungsgemäßen Verfahren wird aus dem Einsatz-$C_4$-Kohlenwasserstoffgemisch zunächst durch Extraktivdestillation mittels des selektiven Lösungsmittels ein Roh-1,3-Butadien abgetrennt, zweckmäßig indem das $C_4$-Kohlenwasserstoffgemisch in ein die im selektiven Lösungsmittel weniger löslichen Kohlenwasserstoffe als 1,3-Butadien, z.B. die Butane, die n-Butene, Isobuten, enthaltendes Raffinat, in einen die im selektivem Lösungsmittel leichter löslichen Kohlenwasserstoffe als 1,3-Butadien, z.B. Butenin, 1-Butin, gegebenenfalls 1,2-Butadien, enthaltenden Strom sowie den Roh-1,3-Butadien-Strom aufgetrennt wird.

Die Extraktivdestillation kann unter Anwendung einer Extraktivdestillationszone durchgeführt werden. Mit besonderem Vorteil wird das Verfahren zur Gewinnung von 1,3-Butadien jedoch unter Anwendung von zwei hintereinandergeschalteten Extraktivdestillationszonen unter Verwendung des gleichen selektiven Lösungsmittels durchgeführt. Hierbei wird beispielsweise in der ersten Extraktivdestillationszone das die weniger löslichen Kohlenwasserstoffe enthaltende Raffinat und ein 1,3-Butadien und die leichter löslichen Kohlenwasserstoffe enthaltender Strom erhalten. Dieser Strom wird anschließend in der zweiten Extraktivdestillationszone in den Roh-1,3-Butadien-Strom, der als Destillat erhalten wird, und einen die leichter löslichen Kohlenwasserstoffe enthaltenden Strom aufgetrennt. Das aus der Extraktivdestillation erhaltene Roh-1,3-Butadien, das als Verunreinigungen leichter als 1,3-Butadien siedende Verunreinigungen wie Propin und höher als 1,3-Butadien siedende Verunreinigungen wie $C_5$-Kohlenwasserstoffe, 1,2-Butadien und gegebenenfalls cis-2-Buten enthält, wird anschließend in zwei zusammengeschalteten Destillationszonen einer destillativen Reinigung unterworfen. Im allgemeinen werden dafür zwei zusammengeschaltete Destillationskolonnen verwendet. Zweckmäßig weist jede Destillationszone eine eigene Wärmezuführung, zweckmäßig durch einen Aufkocher bzw. Verdampfer, und einen eigenen Kondensator auf.

Die erste Destillationszone wird zweckmäßig bei einem Druck von 3 bis 5,5 bar, vorzugsweise 3,5 bis 5 bar, insbesondere 4 bis 4,5 bar betrieben. Die zweite Destillationszone wird im allgemeinen bei einem Druck zwischen 5,5 und 8,5 bar, vorzugsweise zwischen 6 bis 8 bar, insbesondere zwischen 7 bis 7,5 bar betrieben. Im allgemeinen weisen die Destillationszonen eine theoretische Trennstufenzahl von 30 bis 65 Böden, vorzugsweise 35 bis 50 Böden auf.

Das aus der Extraktivdestillation erhaltene Roh-1,3-Butadien wird der ersten Destillationszone dampfförmig zugeführt. Im allgemeinen erfolgt die Zuführung in einem Bereich, der von den beiden oberen Dritteln, vorzugsweise vom mittleren Drittel der ersten Destillationszone gebildet wird. Aus der ersten Destillationszone wird ein 1,3-Butadien und leichtersiedende Verunreinigungen enthaltendes Kopfprodukt flüssig abgezogen und dem mittleren Bereich der zweiten Destillationszone zugeführt, der zweckmäßig von den beiden oberen Dritteln, vorzugsweise von der oberen Hälfte der zweiten Destillationszone gebildet wird. Im Abtriebsteil der ersten Destillationszone, d.h. im Bereich unterhalb der Zuführungsstelle für das Roh-1,3-Butadien, wird ein flüssiger Seitenstrom, der 1,3-Butadien sowie höhersiedende Verunreinigungen enthält, abgezogen und am Sumpf der zweiten Destillationszone zugeführt. Weiter wird das Sumpfprodukt der zweiten Destillationszone, das 1,3-Butadien und höhersiedende Verunreinigungen. enthält, flüssig abgezogen und in die erste Destillationszone im Bereich der Entnahme des flüssigen Seitenstromes zurückgeführt, beispielsweise in einem Bereich, der sich bis zu 5 Böden, vorzugsweise bis zu 2 Böden, oberhalb bzw. vorteilhaft unterhalb der Entnahmestelle des flüssigen Seitenstromes erstreckt. Der Abzug der höhersiedenden Verunreinigungen aus dem System erfolgt am Sumpf der ersten Destillationszone. Die leichtersiedenden Verunreinigungen werden am Kopf der zweiten Destillationszone abgezogen. Das gewünschte 1,3-Butadien-Produkt schließlich wird im Abtriebsteil der zweiten Destillationszone, d.h. im Bereich unterhalb der Zuführungsstelle für das Kopfprodukt der ersten Destillationszone, flüssig entnommen.

Das der destillativen Reinigung dampfförmig zugeführte Roh-1,3-Butadien wird aus der Extraktivdestillation vorteilhaft in der Weise erhalten, daß das Roh-1,3-Butadien aus der Extraktivdestillation als Kopfprodukt abgezogen und einer Teilkondensation unterworfen sind, wobei das Kondensat der Teilkondensation als Rücklauf in die Extraktivdestillation zurückgeführt wird und das bei der Teilkondensation dampfförmig verbleibende Roh-1,3-Butadien, der ersten Destillationszone zugeführt wird. Durch diese Arbeitsweise, bei der anstelle der bei dem bekannten Verfahren durchgeführten Totalkondensation des Roh-1,3-Butadiens eine Teilkondensation durchgeführt wird, kann etwa 75 % der Kühlleistung im Kondensator und die entsprechende Kondensatorfläche eingespart werden, so daß sich hierdurch eine weitere Senkung der Energiekosten und Apparatekosen ergibt.

Nach dem erfindungsgemäßen Verfahren wird ein hochreines 1,3-Butadien erhalten, das hervorragend für die Herstellung von Copolymeren und Polymeren wie Polybutadien geeignet ist.

Das nachstehende Beispiel und Vergleichsbeispiel dienen der weiteren Erläuterung der Erfindung.

Beispiel

Das aus einer Extraktivdestillation erhaltene Zulaufgemisch von Roh-1,3-Butadien, bestehend aus 61,512 kg/h 1,3-Butadien, 0,206 kg/h Propin, 0,008 kg/h trans-2-Buten, 0,205 kg/h cis-2-Buten, 0,117 kg/h 1,2-Butadien, 0,004 kg/h 1-Butin und 0,102 kg/h anderen höhersiedenden Verunreinigungen (im wesentlichen $C_5$-Kohlenwasserstoffe) wird (vgl. Fig.) über Leitung 1 dem mittleren Bereich einer insgesamt 45 theoretische Böden aufweisenden und bei 4,2 bar betriebenen Destillationskolonne 2 ("erste Destillationskolonne") dampfförmig zugeführt. Am Kopf der ersten Destillationskolonne, die mit einem Kondensator 3 und Verdampfer 4 ausgestattet ist, werden bei einer Temperatur von 39° C und einem Rücklaufverhältnis von 2,8 47,11 kg/h eines Stromes, der 99,4 Gew.% 1,3-Butadien und leichtersiedende Verunreinigungen enthält, abgezogen und über Leitung 5 dem mittleren Bereich einer weiteren, bei 7 bar betriebenen Destillationskolonne 6 ("zweite Destillationskolonne") zugeführt, die ebenso wie die erste Destillationskolonne 45 theoretische Böden aufweist und mit dem Kondensator 10 und dem Verdampfer 11 ausgestattet ist. Auf Höhe des 15. theoretischen Bodens der ersten Destillationskolonne wird flüssig ein Seitenstrom von 116,45 kg/h, der 96,9 Gew.% 1,3-Butadien sowie höhersiedende Verunreinigungen enthält, abgezogen und über Leitung 7 im Sumpf der zweiten Destillationskolonne eingespeist. Am Sumpf der ersten Destillationskolonne werden über Leitungen 8 und 9 die höhersiedenden Verunreinigungen in einem Strom von 0,422 kg/h, der noch 0,127 kg/h 1,3-Butadien enthält, abgezogen. Das bei einer Temperatur von 47° und einem Rücklaufverhältnis von 140 dampfförmig über Leitung 12 entnommene Kopfprodukt der zweiten Destillationskolonne von 0,482 kg/h enthält die leichtersiedenden Verunreinigungen und daneben 0,277 kg/h 1,3-Butadien. Der Sumpfaustrag der zweiten Destillationskolonne von 101,83 kg/h, der 99,6 Gew.% 1,3-Butadien und daneben höhersiedende Verunreinigungen enthält, wird flüssig über Leitung 13 zur ersten Destillationskolonne zurückgeführt und dort auf der Höhe der Seitenentnahme eingeleitet. Das Wertprodukt 1,3-Butadien wird aus der zweiten Destillationskolonne auf Höhe des 18. theoretischen Bodens über Leitung 14 mit einer Reinheit von 99,77 % flüssig abgezogen. Bei der ersten Destillationskolonne beträgt die zugeführte Heizleistung 10,65 kW, bei der zweiten Destillationskolonne 9,41 kW, woraus sich eine Gesamtheizleistung von 20,06 kW ergibt.

Vergleichsbeispiel

In einem Vergleichsversuch wird die destillative Reinigung des Roh-1,3-Butadiens in herkömmlicher Weise in zwei Destillationskolonnen mit einer Trennstufenzahl von jeweils 50 theoretischen Böden durchgeführt, indem das Zulaufgemisch von Roh-1,3-Butadien, das in Menge und Konzentration dem Zulaufgemisch vom Beispiel entspricht, flüssig der ersten Destillationskolonne, die bei einem Druck von 7 bar betrieben wird, zugeführt wird, in der die leichtersiedenden Verunreinigungen im Kopfprodukt abgezogen werden. 1,3-Butadien und die höhersiedenden Verunreinigungen werden als Sumpfprodukt entnommen und einer zweiten bei 4,2 bar betriebenen Destillationskolonne zugeführt, in der das Wertprodukt 1,3-Butadien als Kopfprodukt erhalten wird, während die höhersiedenden Verunreinigungen im Sumpfprodukt abgezogen werden.

Um im Vergleichsversuch das Wertprodukt 1,3-Butadien in der gleichen Reinheit wie im Beispiel zu erhalten, ist es erforderlich, Destillationskolonnen mit gegenüber dem Beispiel erhöhter Trennstufenzahl zu verwenden. Die Heizleistung der ersten Destillationskolonne beträgt 8,47 kW, die der zweiten 18,33 kW, woraus sich eine Gesamtheizleistung von 26,8 kW ergibt. Bei der destillativen Reinigung in herkömmlicher Weise gemäß dem Vergleichsversuch ist daher eine um mehr als ein Drittel höhere Gesamtheizleistung erforderlich als bei dem erfindungsgemäßen Verfahren.

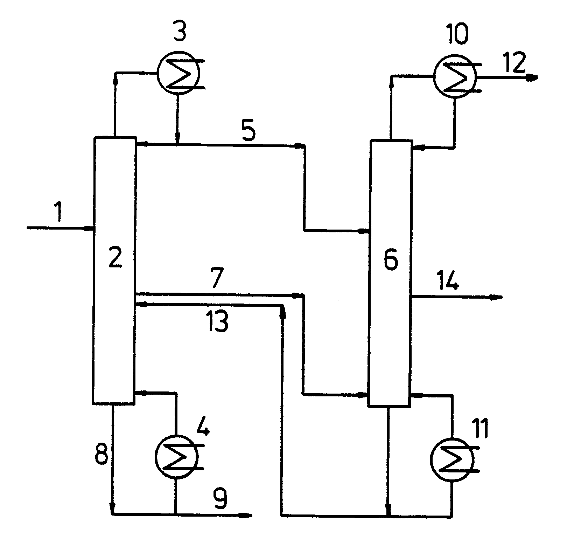

**Patentansprüche**

1. Verfahren zur Gewinnung von 1,3-Butadien aus einem 1,3-Butadien und geringe Mengen Propin und C$_5$-Kohlenwasserstoffe enthaltenden C$_4$-Kohlenwasserstoffgemisch, wobei man zunächst durch Extraktivdestillation mittels eines selektiven Lösungsmittels ein Roh-1,3-Butadien abtrennt, aus dem durch anschließende Destillation unter Abtrennung eines die leichter als 1,3-Butadien siedenden Kohlenwasserstoffe (im folgenden als "leichtersiedende Verunreinigungen" beziechnet) enthaltenden Stromes und eines die höher als 1,3-Butadien siedenden Kohlenwasserstoffe (im folgenden als "höhersiedende Verunreinigungen" bezeichnet) enthaltenden Stromes ein 1,3-Butadien hoher Reinheit erhalten wird, welches dadurch gekennzeichnet ist, daß die Destillation des Roh-1,3-Butadiens in zwei zusammengeschalteten Destillationszonen durchgeführt wird, wobei man

    a) das Roh-1,3-Butadien dampfförmig der ersten Destillationszone zuführt,

    b) aus der ersten Destillationszone ein Kopfprodukt, das 1,3-Butadien sowie leichtersiedende Verunreinigungen enthält, flüssig abzieht und dem mittleren Bereich der zweiten Destillationszone zuführt,

    c) im Abtriebsteil der ersten Destillationszone einen flüssigen Seitenstrom, der 1,3-Butadien sowie höhersiedende Verunreinigungen enthält, abzieht und am Sumpf der zweiten Destillationszone zuführt,

    d) am Sumpf der ersten Destillationszone die höhersiedenden Verunreinigungen abzieht,

    e) am Kopf der zweiten Destillationszone die leichtersiedenden Verunreinigungen abzieht,

    f) im Abtriebsteil der zweiten Destillationszone 1,3-Butadien als Produkt flüssig abzieht und

    g) das Sumpfprodukt der zweiten Destillationszone, das 1,3-Butadien und höhersiedende Verunreinigungen enthält, flüssig abzieht und in die erste Destillationszone im Bereich der Entnahme des flüssigen Seitenstromes zurückgeführt.

2. Verfahren nach Anspruch 1, dadurch gekennzeichnet, daß die erste Destillationszone bei einem Druck von 3 bis 5,5 bar und die zweite Destillationszone bei einem Druck zwischen 5,5 und 8,5 bar betrieben wird.

3. Verfahren nach Anspruch 1 und 2, dadurch gekennzeichnet, daß die Destillationszonen eine theoretische Trennstufenzahl von 30 bis 65 Böden aufweisen.

4. Verfahren nach Anspruch 1 bis 3, dadurch gekennzeichnet, daß das der ersten Destillationszone zugeführte dampfförmige Roh-1,3-Butadien in der Weise erhalten wird, daß Roh-1,3-Butadien aus der Extraktivdestillation als Kopfprodukt abgezogen und einer Teilkondensation unterworfen wird, wobei das Kondensat der Teilkondensation als Rücklauf in die Extraktivdestillation zurückgeführt wird und das bei der Teilkondensation dampfförmig verbleibende Roh-1,3-Butadien, der ersten Destillationszone zugeführt wird.

**Claims**

1. A process for isolating 1,3-butadiene from a C$_4$-hydrocarbon mixture containing 1,3-butadiene and small amounts of propyne and C$_5$-hydrocarbons by first separating off a crude 1,3-butadiene by extractive distillation with a selective solvent and subjecting said crude 1,3-butadiene to a subsequent distillation to separate off a stream containing hydrocarbons having a boiling point lower than that of 1,3-butadiene (hereinafter referred to as lower-boiling impurities) and a stream containing the hydrocarbons having a higher boiling point than 1,3-butadiene (hereinafter referred to as higher-boiling impurities) and obtain a 1,3-butadiene of high purity, said distillation of said crude 1,3-butadiene being carried out in two hooked-up distillation zones, wherein

    a) the crude 1,3-butadiene is fed in vapor form into the first distillation zone,

    b) an overhead product containing 1,3-butadiene and lower-boiling impurities is withdrawn in liquid form from the first distillation zone and fed into the middle section of the second distillation zone,

    c) a liquid sidestream containing 1,3-butadiene and higher-boiling impurities is withdrawn in the stripping portion of the first distillation zone and fed to the base of the second distillation zone,

d) the higher-boiling impurities are withdrawn at the base of the first distillation zone,

e) the lower-boiling impurities are withdrawn at the top of the second distillation zone,

f) 1,3-butadiene is withdrawn in liquid form as the product in the stripping portion of the second distillation zone and

g) the bottom product of the second distillation zone, containing 1,3-butadiene and higher-boiling impurities, is withdrawn in liquid form and returned into the first distillation zone in the section where the liquid sidestream was withdrawn.

2. A process as claimed in claim 1, wherein the first distillation zone is operated under a pressure of from 3 to 5.5 bar and the second distillation zone under a pressure from 5.5 to 8.5 bar.

3. A process as claimed in claim 1 or 2, wherein the distillation zones have from 30 to 65 theoretical plates.

4. A process as claimed in any of claims 1 to 3, wherein the crude 1,3-butadiene fed in vapor form into the first distillation zone is obtained by withdrawing crude 1,3-butadiene from the extractive distillation as overhead product and subjecting it to a partial condensation, the condensate of the partial condensation being returned as reflux into the extractive distillation and the crude 1,3-butadiene remaining in vapor form in the partial condensation being fed into the first distillation zone.

## Revendications

1. Procédé de récupération de 1,3-butadiène à partir d'un mélange d'hydrocarbures en $C_4$ contenant du 1,3-butadième et de petites quantités de propyne et d'hydrocarbures en $C_6$, procédé dans lequel on commence par séparer, par distillation extractive au moyen d'un solvant sélectif, un 1,3-butadiène brut à partir duquel un 1,3-butadiène de haute pureté est obtenu par distillation subséquente avec séparation d'un courant contenant les hydrocarbures de plus bas point d'ébullition que le 1,3-butadiène (désignés ci-après par "impuretés de bas point d'ébullition) et d'un courant contenant les hydrocarbures de plus haut point d'ébullition que le 1,3-butadiène (désignés ci-après par "impuretés de point d'ébullition élevé"), caractérisé en ce qu'on effectue la distillation du 1,3-butadiène brut dans deux zones de distillation accouplées, en procédant

a) en envoyant le 1,3-butadiène brut sous forme de vapeur dans la première zone de distillation,

b) en extrayant à l'état liquide de la première zone de distillation un produit de tête qui contient du 1,3-butadiène ainsi que des impuretés de bas point d'ébullition et en l'envoyant dans la région moyenne de la seconde zone de distillation,

c) en extrayant, dans la partie de prélèvement de la première zone de distillation, un courant latéral liquide qui contient du 1,3-butadiène ainsi que des impuretés de point d'ébullition élevé, et en l'envoyant au bas de la seconde zone de distillation,

d) en extrayant, au bas de la première zone de distillation, les impuretés de point d'ébullition élevé,

e) en extrayant, en tête de la deuxième zone de distillation, les impuretés de bas point d'ébullition,

f) en extrayant à l'état liquide, dans la partie de prélèvement de la seconde zone de distillation, du 1,3-butadiène en tant que produit et

g) en extrayant à l'état liquide le produit du bas de la seconde zone de distillation, qui contient du 1,3-butadiène et des impuretés de point d'ébullition élevé, et en le renvoyant dans la première zone de distillation dans la région du prélèvement du courant latéral liquide.

2. Procédé selon la revendication 1, caractérisé en ce que la première zone de distillation est exploitée sous une pression de 3 à 5,5 bars et la seconde zone de distillation sous une pression comprise entre 5,5 et 8,5 bars.

3. Procédé selon la revendication 1 ou 2, caractérisé en ce que les zones de distillation présentent un nombre d'étages de séparation théoriques de 30 à 65 plateaux.

4. Procédé selon l'une quelconque des revendications 1 à 3, caractérisé en ce que le 1,3-butadiène brut sous forme de vapeur envoyé dans la première zone de distillation est obtenu en extrayant du 1,3-butadiène brut en tant que produit de tête de la distillation extractive et en le soumettant à une condensation partielle, le condensat de la condensation partielle étant renvoyé en tant que produit de recyclage dans la distillation extractive et le 1,3-butadiène qui reste sous forme de vapeur dans la condensation partielle étant envoyé dans la première zone de distillation.